# EUROPEAN PATENT SPECIFICATION

(11) **EP 4 240 297 B1**
(45) Date of publication and mention of the grant of the patent: **03.12.2025**
(21) Application number: 21807470.6
(22) Date of filing: 26.10.2021
(51) Int. Cl.: A61F 5/453

(54) **MALE EXTERNAL CATHETER INCLUDING NONWOVEN FABRIC**
EXTERNER KATHETER FÜR MÄNNER MIT VLIESSTOFF
CATHÉTER EXTERNE MÂLE COMPRENANT UN TEXTILE NON TISSÉ

(30) Priority: 03.11.2020 US 202063109066 P
(43) Date of publication of application: 13.09.2023
(73) Proprietor: Purewick Corporation, El Cajon, California 92020 (US)
(72) Inventor: YIN, Zhihui, Lilburn, Georgia (US); ANDERSON, Michael, Monroe, Georgia 30655 (US); RAMOS RODRIGUEZ, Maria Renee, Florham Park, New Jersey 07932 (US)
(74) Representative: Hoffmann Eitle
(86) International application number: PCT/US2021/056566
(87) International publication number: WO 2022/098536

(56) References cited:
- WO-A1-2019/212949
- WO-A1-2019/212956
- WO-A1-2021/155206
- DE-A1- 2 742 298

## Description

### CROSS-REFERENCE TO RELATED APPLICATIONS

This application claims priority to U.S. Provisional Patent Application No. 63/109,066 filed on November 3, 2020.

### BACKGROUND

A person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, urinary catheters can be uncomfortable, painful, and can lead to complications, such as infections. Additionally, bed pans, which are receptacles used for the toileting of bedridden patients are sometimes used. However, bedpans can be prone to discomfort, spills, and other hygiene issues. WO 2019/212956 A1 discloses fluid collection devices that include a fluid impermeable barrier and a fluid permeable body. The fluid impermeable barrier at least partially defines a chamber and includes an opening extending therethrough. The opening is configured to be positioned adjacent to or receive therein a urethra of a subject. The fluid permeable body has a singular porous material in a substantially cylindrical shape and positioned at least partially within the chamber. WO 2019/212949 A1 discloses systems, devices, and methods for removing fluid from a fluid collection device using a portable vacuum source operably coupled thereto. The fluid collection devices include urine collection devices shaped to complement the female or male anatomy near the respective urethras and the vacuum source is operably coupled to the fluid collection device via one or more sections of conduit.

### SUMMARY

Embodiments of fluid collection devices and systems are disclosed herein. In an embodiment, a fluid collection device includes a sheath at least partially defining a chamber sized and dimensioned to receive at least a portion of a penis of a user therein. The sheath includes a proximal end region defining an opening configured to receive at least a urethral opening of a penis and a distal end region opposite to the proximal end region. The sheath also includes a first layer and a second layer. The first layer includes at least a nonwoven fabric extending at least partially between the proximal end region and the distal end region. The first layer includes a first inner portion and a first outer portion. The second layer includes a fluid permeable multilayer fabric at least partially positioned between the first layer and the chamber and extending at least partially between the proximal end region and the distal end region. The fluid permeable multilayer fabric includes a second inner portion, a second outer portion, and an intermediate portion connecting the second inner portion and the second outer portion. The fluid collection device also includes a head positioned at the distal end region of the sheath. The head defines an aperture proximate to the distal end region configured to be fluidly coupled to a tube effective to provide fluid communication between the chamber and the tube.

In an embodiment, a method of manufacturing a fluid collection device includes securing a first layer including at least a nonwoven fabric to a second layer including at least a fluid permeable multilayer fabric to form a sheath at least partially defining a chamber sized and dimensioned to receive at least a portion of a penis of a user therein. The sheath includes a proximal end region defining an opening configured to receive at least a urethral opening of a penis and a distal end region opposite to the proximal end region. The first layer extends at least partially between the proximal end region and the distal end region and includes a first inner portion and a first outer portion. The second layer is at least partially positioned between the first layer and the chamber and extends at least partially between the proximal end region and the distal end region. The fluid permeable multilayer fabric includes a second inner portion, a second outer portion, and an intermediate portion connecting the second inner portion and the second outer portion. The method also includes securing a head to the distal end region of the sheath. The head defines an aperture proximate to the distal end region configured to be fluidly coupled to a tube effective to provide fluid communication between the chamber and the tube.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is an isometric view of a fluid collection device, according to an embodiment.
**FIG. 2** is a front isometric view of the fluid collection device of **FIG. 1** worn on a male user, according to an embodiment.
**FIG. 3** is a cross-sectional view of the fluid collection device of **FIG. 1****,** taken along line 3-3.
**FIG. 4** is a flow diagram of a method to collect fluids, according to an embodiment.
**FIG. 5** is a block diagram of a fluid collection system, according to an embodiment.
**FIG. 6** is a cross-sectional view of a portion of a sheath of a fluid collection device, according to an embodiment.
**FIG. 7** is a cross-sectional view of a portion of a sheath of a fluid collection device, according to an embodiment.
**FIG. 8** is a cross-sectional view of a portion of a sheath of a fluid collection device, according to an embodiment.
**FIG. 9** is a cross-sectional view of a portion of a sheath of a fluid collection device, according to an embodiment.
**FIG. 10** is a cross-sectional view of a portion of a sheath of a fluid collection device, according to an embodiment.
**FIG. 11** is a flow diagram of a method of manufacturing a fluid collection device, according to an embodiment.

### DETAILED DESCRIPTION

Embodiments of fluid collection devices, systems, and methods of using the same are disclosed herein. An example fluid collection device includes a male-external catheter fluid collection device configured to cover users in the hospital or at home. In some embodiments, the fluid collection devices include a one-piece device having a sheath connected to a base skirt that includes an adhesive. In at least one, some, or all of the embodiments disclosed herein, the fluid collection devices provide the technical effect resulting in use with a user having either a protruding penis or a buried penis in which the penis is covered by excess skin in the pubic area or scrotum. The sheath of the fluid collection device may be shaped and sized to have a male urethra positioned therethrough (*e.g.,* receive a penis therein) and/or be positioned over a buried penis. Whether the penis of the user is protruding or buried, the base skirt may form a seal against the user effective to prevent undesirable leaking of discharged fluids from the fluid collection device. In some embodiments, the sheath may include a layer of one or more nonwoven materials and a layer of three-dimensional (3D) fabric. The fluid collection device also is configured to secure to a conduit to connect the fluid collection device to a vacuum source for removal of discharged fluids from the fluid collection device.

In at least one, some, or all of the embodiments disclosed herein, the nonwoven materials in the sheath may provide venting properties to the fluid collection device that results in the technical effect of allowing a vacuum pressure of about 0 mmHg to about 20 mmHg may be maintained on the fluid collection device for an extended period of time to withdraw fluids (*e.g.,* urine) from the fluid collection device. In at least one, some, or all of the embodiments disclosed herein, the nonwoven material may include and inner surface of the sheath defining a chamber, providing the technical effect of skin contact comfortability to areas around penis of the user. In some embodiments, the nonwoven material may include a multilayer or multiportion material having a fibers or filaments bonding or securing fabric sheets or web structures.

In at least one, some, or all of the embodiments disclosed herein, the 3D fabric may provide the technical effect of keeping the sheath dry during application and use of the fluid collection device, such that embodiments of the fluid collection device may remain in place on the user for up to about 3 to about 5 days. Accordingly, in some embodiments, the 3D fabric may include a fluid permeable multilayer or multiportion fabric including a spacer material between two spaced apart layers. In at least one, some, or all of the embodiments disclosed herein, the 3D fabric may at least temporarily capture and/or direct fluid (*e.g.,* urine) from the user and result in the technical effect of allowing fluid to flow to a conduit for removal from the urine collection device. In at least one, some, or all of the embodiments disclosed herein, the 3D fabric also may provide the technical effect of supporting the urine collection device in a predetermined shape, generally cylindrical, even under application of a vacuum to the interior region of the urine collection device. In at least one, some, or all of the embodiments disclosed herein, the 3D fabric also may prevent the sheath from kinking, resulting in the technical effect of preventing fluid removal from being restricted due to sheath kinking under application of the vacuum source. Accordingly, embodiments of the fluid collection device disclosed herein are suitable for use with a user in any one of a standing, sitting, or supine position. Exemplary fluid collection devices for use with the systems and methods herein are described in more detail below.

The fluid collection devices disclosed herein are configured to collect fluids from an individual. The fluids collected by the fluid collection devices may include urine, penile discharge, reproductive fluids, blood, sweat, or other bodily fluids. Fluid collection devices described herein may be used in fluid collection systems. The fluid collection systems may include a fluid collection device, a fluid storage container, and a stationary or portable vacuum source. Fluid (*e.g.,* urine or other bodily fluids) collected in the fluid collection device may be removed from the fluid collection device via a conduit in fluid communication with the fluid collection device. For example, a first open end of the conduit may extend at least partially into or be connected to the fluid collection device. The second open end of the conduit may extend into or be connected with a fluid collection container or the portable vacuum source. The suction force may be introduced into the interior region of the fluid collection device via the first open end of the conduit responsive to a suction (*e.g*., vacuum) force applied at the second end of the conduit. The suction force may be applied to the second open end of the conduit by the portable vacuum source either directly or indirectly. In some embodiments, the portable vacuum source may be disposed in or on the fluid collection device. In such embodiments, the conduit may extend from the fluid collection device and attach to the portable vacuum source at a first point therein. An additional conduit may attach to the portable vacuum source at a second point thereon and may extend out of the fluid collection device, and may attach to the fluid storage container. Accordingly, a vacuum (*e.g*., suction) may be drawn through the fluid collection device via the fluid storage container. Fluid, such as urine, may be drained from the fluid collection device using the portable vacuum source.

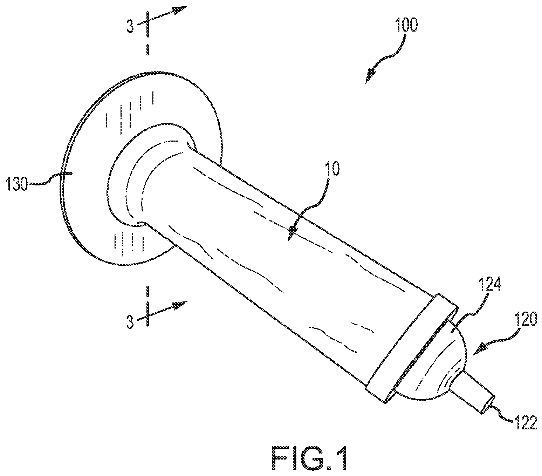

**FIG. 1** is an isometric view of a fluid collection device 100, according to an embodiment. In some embodiments, the fluid collection device 100 includes a sheath 110, a base skirt 130 secured or connected to the sheath 110, and a head 120 secured or connected to the sheath 110 distal to the base skirt 130. The head 120 may include a tapered or bowl-shaped portion 124 and an aperture 122 defined by a port configured to receive or secure to a tube or a conduit.

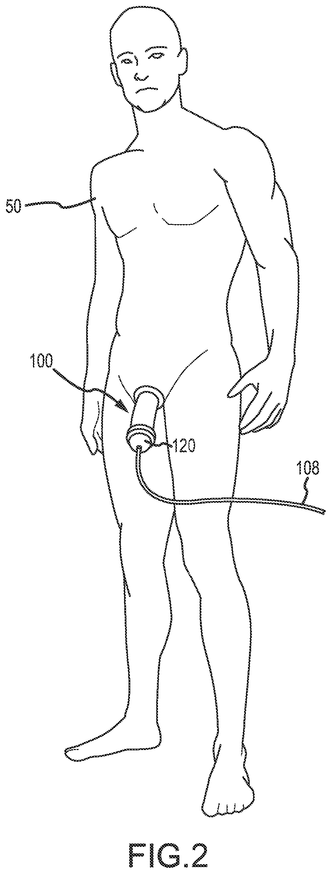

Turning to **FIG. 2****,** which shows the fluid collection device 100 being worn by a user 50, according to an embodiment. The sheath 130 is sized and dimensioned to receive at least a portion of a penis of the user 50 therein. When being worn by the user 50, the fluid collection device 100 may include a tube or conduit 108 detachably or fixedly secured to the head 120 of the fluid collection device 100. The conduit 108 may include a flexible material such as plastic tubing (*e.g*., medical tubing). Such plastic tubing may include a thermoplastic elastomer, polyvinyl chloride, ethylene vinyl acetate, polytetrafluoroethylene, etc., tubing. In some embodiments, the conduit 108 may include silicone or latex. The conduit 108 provides fluid communication between an interior region of the fluid collection device 100, a fluid storage container (not shown), and/or a portable vacuum source (not shown). For example, the conduit 108 may directly or indirectly fluidly couple the interior region of the fluid collection device with the fluid storage container or the portable vacuum source.

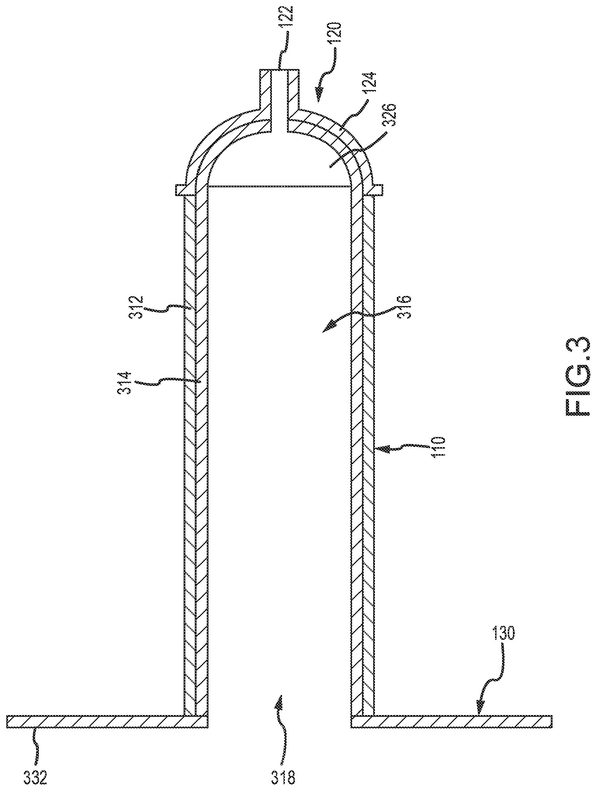

**FIG. 3** is a cross-sectional view of the fluid collection device 100 of **FIG. 1** taken along line 3-3, according to an embodiment. The sheath 110 of the fluid collection device 100 may at least partially define a chamber 316 (interior region) sized and dimensioned to receive at least a portion (*e.g.,* all) of a penis of the user 50 therein. For example, the chamber 316 of the sheath 110 may include a longitudinal length of about 1.3 cm to about 20.3 cm, about 2.5 cm to about 7.6 cm, about 7.6 cm to about 12.7 cm, or about 12.7 cm to about 17.8 cm. In some embodiments, the sheath 110 is generally cylindrical. The sheath 110 also includes a proximal end region and a distal end region opposite to the proximal end region. The proximal end region may define an opening 318 sized and dimensioned to receive at least a urethral opening of a penis and/or at least a portion of the penis of the user 50. In some embodiments, the opening 318 and the chamber 316 may include a substantially equal lateral width or diameter. For example, at least one (*e.g.,* both) of the opening 318 and the chamber 316 may include a lateral width or diameter of about 1.3 cm to about 5 cm.

In some embodiments, the sheath 110 includes at least a first layer 312 (*e.g.,* portion, segment, or region) including at least a nonwoven fabric and a second layer 314 (*e.g*., portion, segment, or region) including a 3D fabric or material. The second layer 314 is at least partially positioned between the first layer 312 and the chamber 316. For example substantially all of the first layer 312 may be spaced from the chamber 316 by the second layer 314. The first layer 312 may be positioned directly adjacent to the second layer 314. In some embodiments, the first layer 312 and the second layer 314 may extend at least partially between the proximal end region and the distal end region of the sheath 110. The first layer 312 and the second layer 314 may be secured together with, for example, an ultrasonic welding, a heat staking, a threading or seam, an adhesive, or combinations thereof. At least one (*e.g.,* both) of the first layer 312 and the second layer 314 may include multiple layers, portions, segments, or regions secured together to form the first layer 312 and/or the second layer 314.

The nonwoven fabric of the first layer 312 may include sheet or web structures bonded together by entangling fiber or filaments and/or by perforating films. The sheet or web structures of the nonwoven fabric layer of the first layer 312 may be bonded together by one or more of mechanical, thermal, or chemical bonding. The nonwoven fabric of the first layer 312 may include flat or tufted porous sheets that are made directly from separate fibers, molten plastic, and/or plastic film. In some embodiments, the nonwoven fabric of the first layer 312 may include on or more of spunbonded polypropylene nonwoven fabric, spunbonded polyester fabric, spunbonded melt blown spunbonded polypropylene nonwoven fabric from BERRY^{™}. The first layer 312 including at least the nonwoven fabric results in the technical effect of providing skin contact comfortability while the fluid collection device 100 is applied. For example, in some embodiments, if the first layer 312 is pressed against the area around the penis or other parts of the body, the nonwoven fabric may provide a comfortable, soft interface against those areas or parts of the body. The first layer 312 including at least the nonwoven fabric also may result in the technical effect of providing venting and/or waterproofing properties to the sheath 110 that allow the fluid collection device 100 to maintain the vacuum condition in a closed system under vacuum operation to withdraw fluids from the fluid collection device 100. Any embodiments of the first layer 312 including at least the nonwoven fabric described herein may be configured to provide venting to the chamber 316 when a vacuum force is exerted or pulled on the chamber 316 through the aperture 122. For example, the first layer 312 may be configured to provided venting to the chamber 316 when a vacuum force of about 0 mmHg to about 30 mmHg is pulled on chamber 316 through the aperture 122, such as a vacuum force of about 0 mmHg to about 15 mmHg, about 15 mmHg to about 30 mmHg, about 0 mmHG to about 10 mmHg, about 10 mmHg to about 20 mmHg, about 20 mmHg to about 30 mmHG, at least about 5 mmHg, at least about 10 mmHg, at least about 15 mmHg, at least about 20 mmHg, at least about 25 mmHg, less than about 5 mmHg, less than about 10 mmHg, less than about 15 mmHg, less than about 20 mmHg, less than about 25 mmHg, or less than about 30 mmHg.

One or more embodiments of the first layer 312 including at least the nonwoven fabric described herein may include pores. The pores may be spread generally uniformly through the first layer 312, or may be substantially isolated in one or more of the portions of the first layer 312a, 312b, 312c, or 312d described in greater detail below. The pores of the first layer 312 may by sized about 0.1 µm to about 5 µm, about 0.1 µm to about 1 µm, about 1 µm to about 2 µm, about 2 µm to about 3 µm, about 3 µm to about 4 µm, about 4 µm to about 5 µm, at least about 0.1 µm, at least about 1 µm, at least about 2 µm, at least about 3 µm, at least about 4 µm, less than about 1 µm, less than about 2 µm, less than about 3 µm, less than about 4 µm, or less than about 5 µm.

In some embodiments, the first layer 312 includes one or more of spunbonded polypropylene and/or melt blown polypropylene. In embodiments, in which the first layer 312 having one or more of spunbonded polypropylene or melt blown polypropylene, the first layer 312 may include a density of about 10 grams per square meter (gsm) to about 30 gsm, about 12 gsm to about 25 gsm, about 12 gsm to about 18.5 gsm, about 18.5 gsm to about 25 gsm, about 10 gsm to about 20 gsm, about 20 gsm to about 30 gsm, about 10 gsm to about 15 gsm, about 15 gsm to about 20 gsm, about 20 gsm to about 25 gsm, about 25 gsm to about 30 gsm, at least about 10 gsm, at least about 15 gsm, at least about 20 gsm, at least about 25 gsm, less than about 15 gsm, less than about 20 gsm, less than about 25 gsm, or less than about 30 gsm. The gsm of the material may include the weight (in grams) of a layer of the material which is one meter by one meter square.

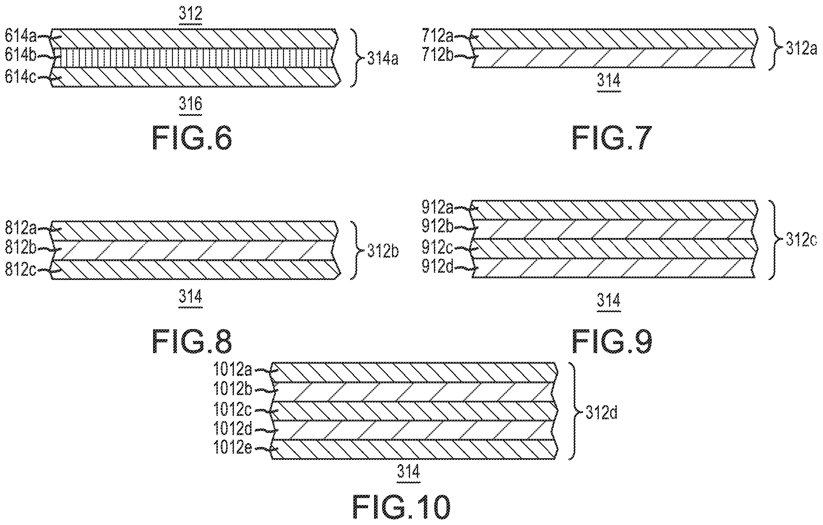

In some embodiments, the first layer 312 including at least the nonwoven fabric may include multiple portions or layers that may be secured to one another to form the first layer 312. Each portion of the first layer may include nonwoven material. For example, turning to **FIG. 7****,** the first layer 312a may include a first outer portion 712a and a first inner portion 712b. In some embodiments, the first inner portion 712b may be adjacent to and/or interface (*e.g*., directly interface) the second layer 314. The first outer portion 712a may define at least a portion of an outer surface of the sheath 110. In some embodiments, an additional layer may cover the first out portion 712a and define at least a portion of the outer surface of the sheath 110. The materials of the first outer portion 712a and the first inner portion 712b of the first layer 312a may vary according to different embodiments. In some examples, the first layer 312a includes a spunbonded fabric including polyethylene and polypropylene. Embodiments of the first layer 312a including the spunbonded fabric including the polyethylene and polypropylene may include the first outer portion 712a and the first inner portion 712b. For example, in some embodiments, the first outer portion 712a includes a polyethylene microporous film and the first inner portion 712b includes a nonwoven polypropylene. The polyethylene microporous film of the first outer portion 712a may include a nonwoven polyethylene microporous film.

Turning to **FIG. 8****,** in some embodiments, the first layer 312b may include a first outer portion 812a, a first inner portion 812c, and a first intermediate portion 812b between (*e.g*., directly between) the first inner portion 812c and the first outer portion 812a. In some embodiments, each of the first inner portion 812c, the first outer portion 812a, and the first intermediate portion 812a may include nonwoven material. In some embodiments, each of the first inner portion 812c and the first outer portion 812a may include nonwoven material. The first inner portion 812c may be adjacent to or interfacing the second layer 314. The first outer portion 812a may define at least a portion of an outer surface of the sheath 110. In some embodiments, an additional layer may cover the first out portion 812a and define at least a portion of the outer surface of the sheath 110. Similar to the first layer 312a, the first layer 312b may include a spunbonded fabric including polyethylene and polypropylene. For example, the first layer 312b may include the first inner portion 812c having a nonwoven polypropylene material, the first outer portion 812a having a polypropylene material (such as a nonwoven polypropylene material), and the first intermediate portion 812b having polyethylene (such as a nonwoven polyethylene) between the first inner portion 812c and the first outer portion 812a.

Embodiments of the first layer 312a or 312b including the spunbonded fabric including the polyethylene and polypropylene also may have a density of about 20 gsm to about 100 gsm, such as about 20 gsm to about 60 gsm, about 60 gsm to about 80 gsm, about 80 gsm to about 100 gsm, about 20 gsm to about 30 gsm, about 30 gsm to about 40 gsm, about 40 gsm to about 50 gsm, about 50 gsm to about 60 gsm, about 60 gsm to about 70 gsm, about 70 gsm to about 80 gsm, about 80 gsm to about 90 gsm, about 90 gsm to about 100 gsm, at least about 20 gsm, at least about 30 gsm, at least about 40 gsm, at least about 50 gsm, at least about 60 gsm, at least 70 gsm, at least about 80 gsm, at least about 90 gsm, at least about 100 gsm, less than about 20 gsm, less than about 30 gsm, less than about 40 gsm, less than about 50 gsm, less than about 60 gsm, less that about 70 gsm, less than about 80 gsm, less than about 90 gsm, or less than about 100 gsm.

Returning to **FIG. 7****,** in some embodiments, materials of the first layer 312a may include a polyester material and a polyethylene material. For example, the first layer 312a may include a first outer portion 712a including a polyethylene material (such as a nonwoven polyethylene material) and a first inner portion including a polyester material (such as a nonwoven polyester material). In embodiments of the first layer 312a including a polyester material and a polyethylene material, the first layer 312a may include a density of about 20 gsm to about 120 gsm, such as about 20 gsm to about 40 gsm, about 40 gsm to about 60 gsm, about 60 gsm to about 80 gsm, about 80 gsm to about 100 gsm, about 100 gsm to about 120 gsm, about 20 gsm to about 30 gsm, about 30 gsm to about 40 gsm, about 40 gsm to about 50 gsm, about 50 gsm to about 60 gsm, about 60 gsm to about 70 gsm, about 70 gsm to about 80 gsm, about 80 gsm to about 90 gsm, about 90 gsm to about 100 gsm, about 100 gsm to about 110 gsm, about 110 gsm to about 120 gsm, at least about 20 gsm, at least about 30 gsm, at least about 40 gsm, at least about 50 gsm, at least about 60 gsm, at least 70 gsm, at least about 80 gsm, at least about 90 gsm, at least about 100 gsm, at least about 110 gsm, at least about 120 gsm, less than about 20 gsm, less than about 30 gsm, less than about 40 gsm, less than about 50 gsm, less than about 60 gsm, less that about 70 gsm, less than about 80 gsm, less than about 90 gsm, less than about 100 gsm, less than about 110 gsm, or less than about 120 gsm.

In some embodiments, materials of the first layer 312a may include spunbonded fabric including a cellulose material and a polyethylene material. For example, the first layer 312a may be spunbonded and include a first outer portion 712a including a polyethylene material and a first inner portion 712b including a cellulose material. In some embodiments, only one of the first outer portion 712a including the polyethylene material and the first inner portion 712b including a cellulose material is spunbonded. In some embodiments, both of the first outer portion 712a including the polyethylene material and the first inner portion 712b including a cellulose material is spunbonded.

Turning again to **FIG. 8****,** in some embodiments, the first layer 312b may include spunbonded polypropylene and melt blown polypropylene. Nonwoven polypropylene fabric may be manufactured through a melt blow process and may, in some example, include only polypropylene. The first outer portion 812a and the first inner portion 812c may both include a spunbonded polypropylene, and the first intermediate portion may include melt blown polypropylene between the first inner portion 812c and the first outer portion 812b. In embodiments of the first layer 312b including spunbonded polypropylene and melt blown polypropylene, the density of the first layer may be about 10 gsm to about 60 gsm, about 10 gsm to about 35 gsm, about 35 gsm to about 60 gsm, about 10 gsm to about 20 gsm, about 20 gsm to about 30 gsm, about 30 gsm to about 40 gsm, about 40 gsm to about 50 gsm, about 50 gsm to about 60 gsm, at least about 10 gsm, at least about 20 gsm, at least about 30 gsm, at least about 40 gsm, at least about 50 gsm, at least about 60 gsm, less than about 20 gsm, less than about 30 gsm, less than about 40 gsm, less than about 50 gsm, or less than about 60 gsm.

In some embodiments, the first layer 312 may include one or more of nonwoven spunbonded polypropylene, nonwoven polyethylene, and/or nonwoven melt blown polypropylene. Turning ahead in the drawings to **FIG. 9****,** according to some embodiments, the first layer 312c may include a first outer portion 912a, a first inner portion 912d, a first intermediate portion 912c, and a second intermediate portion 912b. The first inner portion 912d may be adjacent to or interfacing (*e.g*., directly adjacent to or directly interfacing) the second layer 314. The first outer portion 912a may define at least a portion of an outer surface of the sheath 110. In some embodiments, an additional layer may cover the first out portion 912a and define at least a portion of the outer surface of the sheath 110. The first inner portion 912d may include nonwoven spunbonded polypropylene and the first outer portion 912a may include nonwoven polyethylene. The first intermediate portion 912c may be adjacent to or interfacing (*e.g*., directly adjacent or directly interfacing) to the first inner portion 912d and may include nonwoven melt blown polypropylene. The second intermediate portion 912b may be between (*e.g*., directly between) the first intermediate portion 912c and the first outer portion 912a and may include nonwoven spunbonded polypropylene. The first layer 312c may include a density of about 20 gsm to about 90 gsm, such as about 25 gsm to about 85 gsm, about 20 gsm to about 55 gsm, about 55 gsm to about 90 gsm, about 25 gsm to about 35 gsm, about 35 gsm to about 45 gsm, about 45 gsm to about 55 gsm, about 55 gsm to about 65 gsm, about 65 gsm to about 75 gsm, about 75 gsm to about 85 gsm, at least about 20 gsm, at least about 30 gsm, at least about 40 gsm, at least about 50 gsm, at least about 60 gsm, at least about 70 gsm, at least about 80 gsm, less than about 25 gsm, less than about 35 gsm, less than about 45 gsm, less than about 55 gsm, less than about 65 gsm, less than about 75 gsm, less than about 85, or less than about 95 gsm.

In some embodiments, the first layer 312 may include one or more of nonwoven spunbonded polypropylene, nonwoven polyethylene, and/or nonwoven melt blown polypropylene. Turning ahead in the drawings to **FIG. 10****,** according to some embodiments, the first layer 312d may include a first outer portion 1012a, a first inner portion 1012e, a first intermediate portion 1012d, a second intermediate portion 1012c, and a third intermediate portion 1012b. The first inner portion 1012e may be adjacent to or interfacing (*e.g*., directly adjacent to or directly interfacing) the second layer 314. The first outer portion 1012a may define at least a portion of an outer surface of the sheath 110. In some embodiments, an additional layer may cover the first out portion 1012a and define at least a portion of the outer surface of the sheath 110. The first outer portion 1012a may include nonwoven polyethylene and the first inner portion may include nonwoven spunbonded polypropylene. The first intermediate portion 1012d may be adjacent to or interfacing (*e.g*., directly adjacent to or directly interfacing) the first inner portion 1012e and may include nonwoven melt blown polypropylene. The second intermediate portion 1012c may be adjacent to or interfacing (*e.g*., directly adjacent to or directly interfacing) the first intermediate portion 1012d and may include nonwoven spunbonded polypropylene. The third intermediate portion 1012b may be positioned between (*e.g*., directly between) the second intermediate portion 1012c and the first outer portion 1012a and may include melt blown polypropylene.

Returning in the drawings to **FIG. 3****,** the fluid collection device 100 includes the second layer 314 that may define at least a portion (*e.g*., most or substantially all) of the chamber 316. The second layer 314 may include a 3D or multilayer fluid permeable fabric at least partially positioned between the first layer 312 and the chamber 316. The 3D fabric of the second layer 314 may include two woven fabric layers spaced apart and linked by spacer threads or yarns, such as a fibers. The fibers connecting the two woven fabric layers may include polyester or nylon fibers. The 3D fabric of the second layer 314 may include polyester spacer mesh fabric and/or scuba fabric. The 3D fabric may be fluid permeable and configured to capture fluid (*e.g.,* urine) from the user and/or direct fluid to the conduit 108 for removal from the urine collection device 100. The second layer 314 including the 3D fabric, then, may provide the fluid collection device 100 with urine capture capability and may assist in the drainage or removal of fluids from the urine collection device 100 under a vacuum force. For example, when a user is wearing the urine collection device 100, urine may enter the chamber 316 and then flow through the second layer 314 to the aperture 102 for removal from the urine collection device 100. In some embodiments, the second layer 314 may wick the urine to the aperture 122. The permeable properties referred to herein may be wicking, capillary action, diffusion, or other similar properties or processes, and are referred to herein as "permeable" and/or "wicking." Such "wicking" or other physical properties may exclude absorption second layer 314. While no absorption is desired, the term "substantially no absorption" may allow for nominal amounts of absorption of fluid into the wicking material (*e.g*., absorbency) of the second layer 314, such as less than about 30 wt% of the dry weight of the second layer 314, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the second layer 314. In an embodiment, the second layer 314 may include at least one absorbent or adsorbent material. The second layer 314 may include a one-way fluid movement fabric.

The 3D fabric also may provide the technical effect of supporting the sheath 110 of the urine collection device 100 in a predetermined shape, generally cylindrical. For example, in some embodiments, when a vacuum is applied to the chamber 316 of the sheath 110 via the conduit 108, the 3D fabric of the second layer 314 is configured to support the sheath 110 in a generally cylindrical shape. The second layer 314 including the 3D fabric, then, may provide the technical effect of preventing or inhibiting the fluid restriction that occurs if the sheath kinks when a vacuum is being pulled on the chamber 316. The second layer 314 also may provide relative drying conditions for a user 50, resulting in the technical effect of allowing more long term application of the fluid collection device 100, such as about 3 to about 5 days. Examples of the second layer 314 may include a 3D spacer mesh, a 3D spacer fabric, a spacer fabric, a 3D air spacer mesh, an air spacer fabric, a neoprene scuba fabric, a sandwich scuba fabric, or any combination thereof.

The second layer 314 may include a thickness of about 0.5 mm to about 8 mm, about 1 mm to about 6 mm, about 1.5 mm to about 4.5 mm, about 4.5 mm to about 8 mm, about 1 mm to about 3mm, about 3 mm to about 5 mm, about 5 mm to about 7 mm, at least about 0.5 mm, at least about 1 mm, at least about 2 mm, at least about 3 mm, at least about 4 mm, at least about 5 mm, at least about 6 mm, at least about 7 mm, less than about 1 mm, less than about 2 mm, less than about 3 mm, less than about 4 mm, less than about 5 mm, less than about 6 mm, or less than about 7 mm.

In some embodiments, the second layer 314 may include multiple layers or portions that may be secured to one another. For example, turning ahead in the drawings to **FIG. 6****,** a second layer 314a may include a second outer portion 614a and at least a second inner portion 614c. In more particular examples, the second layer 314a may include the second outer portion 614a, the second inner portion 614c, and an intermediate portion 614b positioned between (*e.g*., directly between) and/or connecting the second inner portion 614c and the second outer portion 614a. In some embodiments, the intermediate portion 614b may include fibers or a fiber material. For example, the fibers of the intermediate portion 614b may include spacer yarns connecting the second outer portion 614a and the second inner portion 614c.

In some embodiments, the second layer 314 includes a spacer fabric configured to capture fluid and help drain the fluid from the urine collection device 100 under a vacuum. The spacer fabric also may act as a supporting material to help hold the shape of the urine collection device 100. The spacer fabric of the second layer 314 may include the second inner portion 614c, the second outer portion 614a, and fibers of the intermediate portion 614b connecting the second inner portion 614c and the second outer portion 614a. In some embodiments, the spacer fabric of the second layer 314 may include a polyester spacer fabric. For example, at least the second inner portion 614c and the second outer portion 614a of the second layer 314 may include and/or consist essentially of a polyester material. In some examples of the second layer 314 including a spacer fabric having polyester or other materials, the second inner portion 614c may include an inner mesh portion having holes or openings of a first size and the second outer portion 614a may include an outer mesh portion having holes of a second size smaller than the holes of the inner mesh portion having the holes of the first size. In some examples of the second layer 314 including a spacer fabric having polyester or other materials, the second inner portion 614c may include a polyester fabric and the second outer portion 614a may include a polyester mesh material having a plurality of holes or openings. In some examples of the second layer 314 including a spacer fabric having polyester or other materials, the second inner portion 614c may include a cotton fabric and the second outer portion may include a polyester mesh having a plurality of holes or openings.

The second layer 314 including the spacer fabric may include a density of about 150 gsm to about 300 gsm, about 190 gsm to about 260 gsm, about 150 gsm to about 190 gsm, about 190 gsm to about 230 gsm, about 230 gsm to about 260 gsm, about 260 gsm to about 300 gsm, about 190 gsm to about 200 gsm, about 200 gsm to about 210 gsm, about 210 gsm to about 220 gsm, about 230 gsm to about 240 gsm, about 240 gsm to about 250 gsm, about 250 gsm to about 260 gsm, at least about 190 gsm, at least about 200 gsm, at least about 210 gsm, at least about 220 gsm, at least about 230 gsm, at least about 240 gsm, at least about 250 gsm, at least about 260 gsm, less than about 190 gsm, less than about 200 gsm, less than about 210 gsm, less than about 220 gsm, less than about 230 gsm, less than about 240 gsm, less than about 250 gsm, or less than about 260 gsm.

In some embodiments, the second layer 314 includes a scuba fabric. The scuba fabric of the second layer 314 may include a neoprene or polychloroprene material. In some embodiments, the scuba fabric includes a polyester-spandex knit fabric. For example, the scuba fabric of the second layer 314 may include about 10 wt% to about 25 wt% neoprene or polychloroprene material. The scuba fabric of the second layer 314 may include one or more of the second inner portion 614c, the second outer portion 614a, or the intermediate portion 614b connecting the second inner portion 614c and the second outer portion 614a. In some embodiments, the second outer portion 614a includes a polyester fabric, the second inner portion 614c includes a polyester fabric, and the intermediate portion 614b includes the scuba fabric including the neoprene. In some embodiments, at least one (*e.g.,* both) of the second outer portion 614a and the second inner portion 614c includes the scuba fabric. The intermediate portion 614b may include fibers such as spacer yarns connecting the second outer portion 614a and the second inner portion 614c when the second outer portion 614a and the second inner portion 614c include the scuba fabric.

In some embodiments, the second outer portion 614a includes a polyester fabric, the second inner portion 614c includes a cotton fabric, and the intermediate portion 614b includes the scuba fabric including the neoprene. In some embodiments, at least one (*e.g.,* both) of the second outer portion 614a and the intermediate portion 614b may include scuba fabric and the second inner portion 614c may include cotton fabric. The intermediate portion 614b may include fibers such as spacer yarns connecting the second outer portion 614a and the second inner portion 614c when the second outer portion 614a includes scuba fabric and the second inner portion 614c includes cotton fabric.

The second layer 314 including the scuba fabric may include a density of about 225 gsm to about 400 gsm, about 250 gsm to about 380 gsm, about 250 gsm to about 300 gsm, about 300 gsm to about 350 gsm, about 350 gsm to about 400 gsm, about 250 gsm to about 260 gsm, about 260 gsm to about 270 gsm, about 270 gsm to about 280 gsm, about 280 gsm to about 290 gsm, about 290 gsm to about 300 gsm, about 300 gsm to about 310 gsm, about 310 gsm to about 320 gsm, about 320 gsm to about 330 gsm, about 330 gsm to about 340 gsm, about 350 gsm, about 350 gsm to about 360 gsm, about 360 gsm to about 370 gsm, about 370 gsm to about 380 gsm, at least about 250 gsm, at least about 260 gsm, at least about 270 gsm, at least about 280 gsm, at least about 290 gsm, at least about 300 gsm, at least about 310 gsm, at least about 320 gsm, at least about 330 gsm, at least about 340 gsm, at least about 350 gsm, at least about 360 gsm, at least about 370 gsm, at least about 380 gsm, less than about 250 gsm, less than about 260 gsm, less than about 270 gsm, less than about 280 gsm, less than about 290 gsm, less than about 300 gsm, less than about 310 gsm, less than about 320 gsm, less than about 330 gsm, less than about 340 gsm, less than about 350 gsm, less than about 360 gsm, less than about 370 gsm, or less than about 380 gsm.

The various embodiments of the first layer 312 and the second layer 314 described herein may be combined without limitation in the sheath 110 of the fluid collection device 100. For example, embodiments of the second layer 314a described herein may be combined with any one or more of the embodiments of the first layer 312a, 312b, 312c, or 312d.

Returning in the drawings to **FIG. 3****,** the fluid collection device 100 also includes a head 120 secured to the distal end region of the sheath 110, according to some embodiments. The head 120 may be formed of any suitable fluid impermeable material(s), such as a fluid impermeable polymer (*e.g*., silicone, polypropylene, polyethylene, polyethylene terephthalate, a polycarbonate, a thermoplastic elastomer, polyvinyl chloride, ethylene vinyl acetate, polytetrafluoroethylene etc.), a metal film, natural rubber, another suitable material, or combinations thereof. The head 120 may at least partially define the aperture 122 configured to receive the tube or conduit 108 effective provide fluid communication between the chamber 316 or other interior region of the fluid collection device 100 and the tube or conduit 108. In some embodiments, the head 120 includes a protruding port sized and dimensioned to be inserted into the tube or conduit 108 to friction fit the head 120 to the tube or conduit 108. The head 120 may be secured to the sheath 110 with, for example, an ultrasonic welding, a heat staking, a threading or seam, an adhesive, or combinations thereof.

In some embodiments, the head 120 may taper between the sheath 110 and the aperture 122. For example, the head 120 may include a semispherical 326 or conical portion tapering at least partially between the sheath 110 and the aperture 122. In some embodiments, the second layer 314 extends at least partially into the region of the head 120. For example, the second layer 314 may be secured to or line an inner surface of the head 120. In some embodiments (not shown), the second layer 314 is positioned between the chamber 316 and the aperture 122. In some embodiments, the second layer 314 includes a gap or opening aligned with the aperture 122 such that the second layer 314 is not positioned between the chamber 316 and the aperture 122.

The fluid collection device 100 also may include a base skirt 130, according to an embodiment. The base skirt 130 may be secured to the proximal end region of the sheath 110. For example, the base skirt 130 may be secured to the proximal end region of the sheath 110 with an ultrasonic welding, a heat staking, a threading or seam, an adhesive, or combinations thereof. In some embodiments, the base skirt 130 is connected directly to the sheath 110. The base skirt 130 may extend at least partially (*e.g*., entirely) around the opening 318. For example, the base skirt 130 may be a generally annular base skirt 130. The base skirt 130 and the sheath 110 are connected such that the when the base skirt 130 is secured to the user 50, the base skirt 130 forms an at least partial seal between the chamber 316 and the exterior, ambient environment of the fluid collection device 100. With the base skirt 130 forming a seal, fluid discharged into the chamber 316 by a urethra either positioned in the chamber or buried on the user 50 beneath the chamber 316 remains in the chamber 316 for removal through the aperture 122.

The base skirt 130 may include a surface having an adhesive 332 configured to adhere the base skirt 130 to the skin of the user 50 around the penis. The base skirt 130 and/or the surface of the base skirt including the adhesive 332 may be positioned such that the adhesive 332 adheres only to the skin of the user 50 around the penis, effective to enable the fluid collection device to cover either a protruding penis or a buried penis. The adhesive 332 may be positioned on the surface of the base skirt 130 generally opposite to the sheath 110 and/or head 120 of the fluid collection device 100. The adhesive 332 may be positioned at least partially (*e.g*., entirely) around the opening 318. The adhesive 332 may include one or more of a silicone gel adhesive, an acrylate/acrylic adhesive, an acrylate/acrylic gel adhesives, a hydrogel adhesive, or any combination thereof. On stainless steel or on low density polyethylene under degree peel testing, the adhesion strength of the adhesive 332 may be about 0.25 lb/in to about 3 lb/in, about 0.4 to about 2.5 lb/in, about 0.4 to about 1 lb/in, about 1 lb/in to about 1.5 lb/in, about 1.5 lb/in to about 2 lb/in, about 2 lb/in to about 2.5 lb/in, at least about 0.4 lb/in, at least about 1 lb/in, at least about 1.5 lb/in, at least about 2 lb/in, at least about 2.5 lb/in, less than about 0.4 lb/in, less than about 1 lb/in, less than about 1.5 lb/in, less than about 2 lb/in, or less than about 2.5 lb/in.

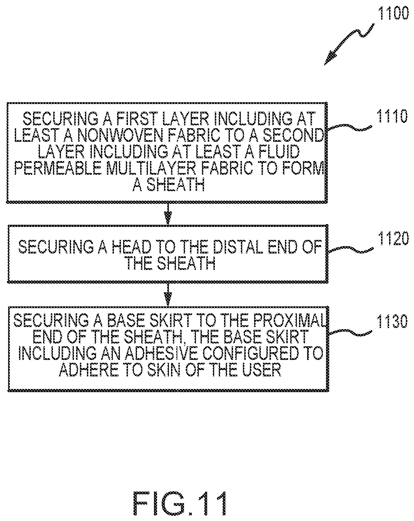

Turning ahead in the drawings to **FIG. 11****,** a method 1100 of manufacturing any of the fluid collection devices described herein is provided. The method 1100 may include an act 1110 securing a first layer including at least a nonwoven fabric to a second layer including at least a fluid permeable multilayer fabric to form a sheath at least partially defining a chamber sized and dimensioned to receive at least a portion of a penis of a user therein. The sheath may include a proximal end region defining an opening configured to receive at least a urethral opening of a penis and a distal end region opposite to the proximal end region. The first layer may extend at least partially between the proximal end region and the distal end region and includes a first inner portion and a first outer portion. The second layer may be at least partially positioned between the first layer and the chamber and may extend at least partially between the proximal end region and the distal end region. The fluid permeable multilayer fabric may include a second inner portion, a second outer portion, and an intermediate portion connecting the second inner portion and the second outer portion. The first layer and the second layer in the method 1100 may include any first layer and second layer described herein.

The method 1100 also includes an act 1120 of securing a head to the distal end region of the sheath, the head defining an aperture proximate to the distal end region configured to be fluidly coupled to a tube effective to provide fluid communication between the chamber and the tube. In some embodiments, the act 1120 includes securing a fluid impermeable barrier of the head to the distal end region of the sheath. More particularly, the act 1120 may include securing the fluid impermeable barrier of the head to the distal end region of the sheath such that the second layer extends at least partially into the head.

In some embodiments, the method 1100 also includes an act 1130 of securing a base skirt to the proximal end region of the sheath, the base skirt including an adhesive configured adhere to skin of the user around the penis. The adhesive may include one or more of a silicone gel adhesive, an acrylate/acrylic adhesive, an acrylate/acrylic gel adhesives, a hydrogel adhesive, or any combination thereof. In some embodiments, the act 1130 includes welding the base skirt to the proximal end region of the sheath.

The acts 1110, 1120, and 1130 of the method 1100 are for illustrative purposes. For example, the acts 1110, 1120, and 1130 of the method 1100 may be performed in different orders, split into multiple acts, modified, supplemented, or combined. In an embodiment, one or more of the acts 1110, 1120, and 1130 of the method 1100 may be omitted from the method 1100. Any of the 1110, 1120, and 1130 of the method 1100 may include using any of the first layers or the second layers disclosed herein.

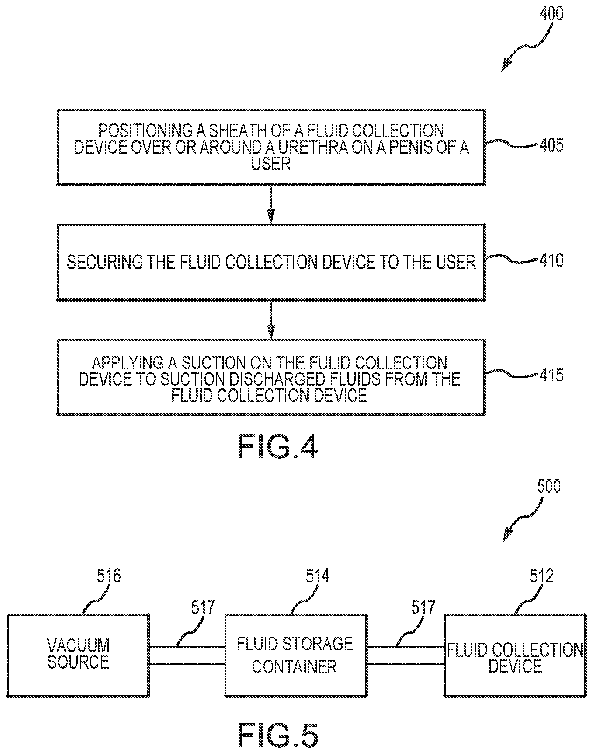

**FIG. 4** is a flow diagram of a method 400 for collecting fluids, according to an embodiment. The method 400 includes an act 405 of positioning a sheath of a fluid collection device over or around a urethra of a penis of the user. The fluid collection device may include any fluid collection device described herein, such as the fluid collection device 100. In some embodiments, the act 405 may include positioning the sheath around the urethra of the penis of the user by inserting at least a portion of the penis into the sheath. In some embodiments, the act 405 includes positioning an opening of the sheath over the urethra of the penis that is buried on the user. The method 400 also includes an act 410 of securing the fluid collection device to the user. The act 410 may include securing adhesive of a base skirt connected to the sheath to at least a portion of skin around the penis of the user. The method 400 also may include an act of receiving fluids from the urethra into the chamber of the fluid collection device. The method 400 also includes an act 415 of applying suction effective to suction the fluids from the chamber via a secured thereto.

Acts 405, 410, and 415 of the method 400 are for illustrative purposes. For example, the acts 405, 410, and 415 of the method 400 may be performed in different orders, split into multiple acts, modified, supplemented, or combined. In an embodiment, one or more of the acts 405, 410, and 415 of the method 400 may be omitted from the method 400. Any of the 405, 410, and 415 of the method 400 may include using any of the fluid collection devices or systems disclosed herein.

**FIG. 5** is a block diagram of a fluid collection system 500, according to an embodiment. The system 500 includes a fluid collection device 512 (*e.g.,* any of the fluid collection devices disclosed herein), a fluid storage container 514, and a vacuum source 516. The fluid collection device 512, the fluid storage container 514, and the vacuum source 516 may be fluidly coupled to each other via one or more conduits 517. For example, fluid collection device 512 may be operably coupled to one or more of the fluid storage container 514 or the vacuum source 516 via the conduit 517. Fluid (*e.g.,* urine or other bodily fluids) collected in the fluid collection device 512 may be removed from the fluid collection device 512 via the conduit 517 secured to the fluid collection device 512. Suction force may be introduced into the chamber of the fluid collection device 512 via the inlet of the conduit 517 responsive to suction (*e.g*., vacuum) force applied at the outlet of the conduit 517.

The suction force may be applied to the outlet of the conduit 517 by the vacuum source 516 either directly or indirectly. The suction force may be applied indirectly via the fluid storage container 514. For example, the outlet of the conduit 517 may be disposed within the fluid storage container 514 and an additional conduit 517 may extend from the fluid storage container 514 to the vacuum source 516. Accordingly, the vacuum source 516 may apply suction to the fluid collection device 512 via the fluid storage container 514. The suction force may be applied directly via the vacuum source 516. For example, the outlet of the conduit 517 may be disposed within the vacuum source 516. An additional conduit 517 may extend from the vacuum source 516 to a point outside of the fluid collection device 512, such as to the fluid storage container 514. In such examples, the vacuum source 516 may be disposed between the fluid collection device 512 and the fluid storage container 514.

The fluid storage container 514 is sized and shaped to retain a fluid therein. The fluid storage container 514 may include a bag (*e.g.,* drainage bag), a bottle or cup (*e.g.,* collection jar), or any other enclosed container for storing bodily fluid(s) such as urine. In some examples, the conduit 517 may extend from the fluid collection device 512 and attach to the fluid storage container 514 at a first point therein. An additional conduit 517 may attach to the fluid storage container 514 at a second point thereon and may extend and attach to the vacuum source 516. Accordingly, a vacuum (*e.g*., suction) may be drawn through fluid collection device 512 via the fluid storage container 514. Fluid, such as urine, may be drained from the fluid collection device 512 using the vacuum source 516.

The vacuum source 516 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 516 may provide a vacuum or suction to remove fluid from the fluid collection device 512. In some examples, the vacuum source 516 may be powered by one or more of a power cord (*e.g.,* connected to a power socket), one or more batteries, or even manual power (*e.g.,* a hand operated vacuum pump). In some examples, the vacuum source 516 may be sized and shaped to fit outside of, on, or within the fluid collection device 512. For example, the vacuum source 516 may include one or more miniaturized pumps or one or more micro pumps. The vacuum sources disclosed herein may include one or more of a switch, a button, a plug, a remote, or any other device suitable to activate the vacuum source 516.

While various aspects and embodiments have been disclosed herein, other aspects and embodiments are contemplated. The various aspects and embodiments disclosed herein are for purposes of illustration and are not intended to be limiting.

Terms of degree (*e.g*., "about," "substantially," "generally," etc.) indicate structurally or functionally insignificant variations. Structurally or functionally insignificant variations are known to a person having ordinary skill in the art. For example, in some embodiments, a person having ordinary skill in the art would understand that structurally or functional insignificant variations may include varying the quantity modified by the term of degree by ± 10%, ±5%, or +2%. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection device (100), comprising:
a sheath (110) at least partially defining a chamber (316) sized and dimensioned to receive at least a portion of a penis of a user therein and including:
a proximal end region defining an opening (318) configured to receive at least a urethral opening of a penis;
a distal end region opposite to the proximal end region;
a first layer (312, 312a, 312b, 312c, 312d) including at least a nonwoven fabric extending at least partially between the proximal end region and the distal end region, the first layer including a first inner portion (712b, 812c, 912d, 1012e) and a first outer portion (712a, 812a, 912a, 1012a); and
a second layer (314, 314a) including a fluid permeable multilayer fabric at least partially positioned between the first layer and the chamber and extending at least partially between the proximal end region and the distal end region, the fluid permeable multilayer fabric including a second inner portion (614c), a second outer portion (614a), and an intermediate portion (614b) connecting the second inner portion and the second outer portion; and
a head (120) positioned at the distal end region of the sheath, the head defining an aperture (122) proximate to the distal end region configured to be fluidly coupled to a tube (108) effective to provide fluid communication between the chamber and the tube.

2. The fluid collection device of claim 1, wherein the first layer includes a spunbonded fabric having the first inner portion and the first outer portion and including polyethylene and polypropylene.

3. The fluid collection device of claim 2, wherein the first inner portion includes a nonwoven polypropylene and the first outer portion includes a polyethylene microporous film.

4. The fluid collection device of claim 2, wherein the first inner portion includes a nonwoven polypropylene material, the first outer portion includes a nonwoven polypropylene material, and the first layer includes a first intermediate portion including polyethylene between the first inner portion and the first outer portion.

5. The fluid collection device of claim 1, wherein the first inner portion includes a nonwoven polyester material and the first outer portion includes a polyethylene material.

6. The fluid collection device of claim 1, wherein the first layer includes a spunbonded fabric, the first inner portion includes a cellulose material and the first outer portion includes polyethylene.

7. The fluid collection device of claim 1, wherein the first inner portion and the first outer portion include spunbonded polypropylene, and the first layer further includes an intermediate portion including melt blown polypropylene between the first inner portion and the first outer portion.

8. The fluid collection device of claim 1, wherein the first inner portion includes spunbonded polypropylene and the first outer portion includes nonwoven polyethylene, the first layer further including a first intermediate portion including melt blown polypropylene adjacent to the first inner portion, a second intermediate portion including spunbonded polypropylene adjacent to the first intermediate portion, and a third intermediate portion including melt blown polypropylene between the second intermediate portion and the first outer portion.

9. The fluid collection device of claim 1, wherein the first inner portion includes spunbonded polypropylene and the first outer portion includes nonwoven polyethylene, the first layer further including a first intermediate portion including melt blown polypropylene adjacent to the first inner portion and a second intermediate portion including spunbonded polypropylene between the first intermediate portion and the first outer portion.

10. The fluid collection device of any of claims 1-9, wherein the first layer is configured to provide venting to the chamber when a vacuum of about 0 mmHg to about 20 mmHg is pulled on the chamber through the aperture.

11. The fluid collection device of any of claims 1-10, wherein the first layer includes a porous layer having pores of about 0.1 µm to about 3 µm; or
wherein the second layer includes a spacer fabric including the second inner portion, the second outer portion, and the intermediate portion connecting the second inner portion and the second outer portion; wherein the spacer fabric of the second layer includes a polyester spacer fabric; or
wherein the second inner portion includes an inner mesh portion having holes of a first size and the second outer portion includes an outer mesh portion having holes of a second size smaller than the holes of the first size; or
wherein the second inner portion includes a polyester fabric and the second outer portion includes polyester mesh; or wherein the second inner portion includes a cotton fabric and the second outer portion includes polyester mesh; or
wherein the spacer fabric has a density of about 190 gsm to about 260 gsm.

12. The fluid collection device of claim 1, wherein the second layer includes a polyester-spandex knit fabric and at least one of:
the second inner portion includes the polyester-spandex knit fabric and the second outer portion includes the polyester-spandex knit fabric; or
the second inner portion includes a cotton fabric and the second outer portion includes the polyester-spandex knit fabric; or
the polyester-spandex knit fabric has a density of about 250 gsm to about 380 gsm.

13. The fluid collection device of any of claims 1-12, wherein the second layer has a thickness of about 1.0 mm to about 6.0 mm.

14. The fluid collection device of any of claims 1-13, wherein the second inner portion of the second layer at least partially defines the chamber.

15. The fluid collection device of any of claims 1-14, wherein the first outer portion of the first layer at least partially defines an outer surface of the sheath.

## Patentansprüche

1. Fluidsammelvorrichtung (100), umfassend:
eine Hülle (110), die zumindest teilweise eine Kammer (316) definiert, die so bemessen und dimensioniert ist, um zumindest einen Abschnitt eines Penis eines Benutzers darin aufzunehmen und einzuschließen:
einen proximalen Endbereich, der eine Öffnung (318) definiert, die so gestaltet ist, dass sie mindestens eine Harnröhrenöffnung eines Penis aufnimmt;
einen distalen Endbereich, der dem proximalen Endbereich gegenüberliegt;
eine erste Schicht (312, 312a, 312b, 312c, 312d), die zumindest einen Vliesstoff einschließt, der sich zumindest teilweise zwischen dem proximalen Endbereich und dem distalen Endbereich erstreckt, wobei die erste Schicht einen ersten inneren Abschnitt (712b, 812c, 912d, 1012e) und einen ersten äußeren Abschnitt (712a, 812a, 912a, 1012a) einschließt; und
eine zweite Schicht (314, 314a), die ein flüssigkeitsdurchlässiges Mehrschichtgewebe einschließt, das zumindest teilweise zwischen der ersten Schicht und der Kammer angeordnet ist und sich zumindest teilweise zwischen dem proximalen Endbereich und dem distalen Endbereich erstreckt, wobei das flüssigkeitsdurchlässige Mehrschichtgewebe einen zweiten inneren Abschnitt (614c), einen zweiten äußeren Abschnitt (614a) und einen Zwischenabschnitt (614b) einschließt, der den zweiten inneren Abschnitt und den zweiten äußeren Abschnitt verbindet; und
einen Kopf (120), der am distalen Endbereich der Hülle positioniert ist, wobei der Kopf eine Apertur (122) in der Nähe des distalen Endbereichs definiert, die konfiguriert ist, um fluidmäßig mit einem Rohr (108) gekoppelt zu werden, um eine Fluidverbindung zwischen der Kammer und dem Rohr herzustellen.

2. Fluidsammelvorrichtung nach Anspruch 1, wobei die erste Schicht ein Spinnvlies mit dem ersten inneren Abschnitt und dem ersten äußeren Abschnitt einschließt und Polyethylen und Polypropylen einschließt.

3. Fluidsammelvorrichtung nach Anspruch 2, wobei der erste innere Abschnitt ein Polypropylenvlies einschließt und der erste äußere Abschnitt eine mikroporöse Polyethylenfolie einschließt.

4. Fluidsammelvorrichtung nach Anspruch 2, wobei der erste innere Abschnitt ein Polypropylen-Vliesmaterial einschließt, der erste äußere Abschnitt ein Polypropylen-Vliesmaterial einschließt und die erste Schicht einen ersten Zwischenabschnitt einschließt, der Polyethylen zwischen dem ersten inneren Abschnitt und dem ersten äußeren Abschnitt einschließt.

5. Fluidsammelvorrichtung nach Anspruch 1, wobei der erste innere Abschnitt ein Polyestervliesmaterial und der erste äußere Abschnitt ein Polyethylenmaterial einschließt.

6. Fluidsammelvorrichtung nach Anspruch 1, wobei die erste Schicht ein Spinnvlies einschließt, der erste innere Abschnitt ein Zellulosematerial und der erste äußere Abschnitt Polyethylen einschließt.

7. Fluidsammelvorrichtung nach Anspruch 1, wobei der erste innere Abschnitt und der erste äußere Abschnitt spinngebundenes Polypropylen einschließen und die erste Schicht weiter einen Zwischenabschnitt einschließt, der schmelzgeblasenes Polypropylen zwischen dem ersten inneren Abschnitt und dem ersten äußeren Abschnitt einschließt.

8. Fluidsammelvorrichtung nach Anspruch 1, wobei der erste innere Abschnitt spinngebundenes Polypropylen einschließt und der erste äußere Abschnitt Polyethylenvlies einschließt, wobei die erste Schicht weiter einen ersten Zwischenabschnitt einschließt, der schmelzgeblasenes Polypropylen angrenzend an den ersten inneren Abschnitt einschließt, einen zweiten Zwischenabschnitt, der spinngebundenes Polypropylen angrenzend an den ersten Zwischenabschnitt einschließt, und einen dritten Zwischenabschnitt, der schmelzgeblasenes Polypropylen zwischen dem zweiten Zwischenabschnitt und dem ersten äußeren Abschnitt einschließt.

9. Fluidsammelvorrichtung nach Anspruch 1, wobei der erste innere Abschnitt spinngebundenes Polypropylen einschließt und der erste äußere Abschnitt Polyethylenvlies einschließt, wobei die erste Schicht weiter einen ersten Zwischenabschnitt einschließt, der schmelzgeblasenes Polypropylen angrenzend an den ersten inneren Abschnitt einschließt, und einen zweiten Zwischenabschnitt, der spinngebundenes Polypropylen zwischen dem ersten Zwischenabschnitt und dem ersten äußeren Abschnitt einschließt.

10. Fluidsammelvorrichtung nach einem der Ansprüche 1-9 wobei die erste Schicht konfiguriert ist, um die Kammer zu entlüften, wenn ein Vakuum von etwa 0 mmHg bis etwa 20 mmHg durch die Apertur an die Kammer gezogen wird.

11. Fluidsammelvorrichtung nach einem der Ansprüche 1-10, wobei die erste Schicht eine poröse Schicht mit Poren von etwa 0,1 µm bis etwa 3 µm einschließt; oder
wobei die zweite Schicht ein Abstandsgewebe einschließt, das den zweiten inneren Abschnitt, den zweiten äußeren Abschnitt und den Zwischenabschnitt, der den zweiten inneren Abschnitt und den zweiten äußeren Abschnitt verbindet, einschließt; wobei das Abstandsgewebe der zweiten Schicht ein Polyester-Abstandsgewebe einschließt; oder
wobei der zweite innere Abschnitt einen inneren Netzgewebeabschnitt mit Löchern einer ersten Größe einschließt und der zweite äußere Abschnitt einen äußeren Netzgewebeabschnitt mit Löchern einer zweiten Größe einschließt, die kleiner sind als die Löcher der ersten Größe; oder
wobei der zweite innere Abschnitt ein Polyestergewebe einschließt und der zweite äußere Abschnitt Polyesternetzgewebe einschließt; oder wobei der zweite innere Abschnitt ein Baumwollgewebe einschließt und der zweite äußere Abschnitt Polyesternetzgewebe einschließt; oder wobei das Abstandsgewebe eine Dichte von etwa 190 g/m² bis etwa 260 g/m² aufweist.

12. Fluidsammelvorrichtung nach Anspruch 1, wobei die zweite Lage ein Polyester-Elasthan-Strickgewebe einschließt und mindestens eines aus Folgenden:
der zweite innere Abschnitt schließt das Polyester-Elasthan-Strickgewebe ein und der zweite äußere Abschnitt schließt das Polyester-Elasthan-Strickgewebe ein; oder
der zweite innere Abschnitt schließt ein Baumwollgewebe ein und der zweite äußere Abschnitt schließt das Polyester-Elasthan-Gewebe ein; oder
das Polyester-Elasthan-Strickgewebe weist eine Dichte von etwa 250 g/m² bis etwa 380 g/m² auf.

13. Fluidsammelvorrichtung nach einem der Ansprüche 1-12, wobei die zweite Schicht eine Dicke von etwa 1,0 mm bis etwa 6,0 mm aufweist.

14. Fluidsammelvorrichtung nach einem der Ansprüche 1-13, wobei der zweite innere Abschnitt der zweiten Schicht zumindest teilweise die Kammer begrenzt.

15. Fluidsammelvorrichtung nach einem der Ansprüche 1-14, wobei der erste äußere Abschnitt der ersten Schicht zumindest teilweise eine Außenfläche der Hülle definiert.

## Revendications

1. Dispositif (100) de collecte de fluide, comprenant :
une gaine (110) délimitant au moins partiellement une chambre (316) taillée et dimensionnée pour recevoir au moins une partie d'un pénis d'un utilisateur à l'intérieur de celle-ci et incluant :
une région d'extrémité proximale définissant une ouverture (318) configurée pour recevoir au moins une ouverture urétrale d'un pénis ;
une région d'extrémité distale opposée à la région d'extrémité proximale ;
une première couche (312, 312a, 312b, 312c, 312d) incluant au moins un textile non tissé s'étendant au moins partiellement entre la région d'extrémité proximale et la région d'extrémité distale, la première couche incluant une première partie (712b, 812c, 912d, 1012e) interne et une première partie (712a, 812a, 912a, 1012a) externe ; et
une seconde couche (314, 314a) incluant un textile multicouche perméable aux fluides positionné au moins partiellement entre la première couche et la chambre et s'étendant au moins partiellement entre la région d'extrémité proximale et la région d'extrémité distale, le textile multicouche perméable aux fluides incluant une seconde partie (614c) interne, une seconde partie (614a) externe et une partie (614b) intermédiaire reliant la seconde partie interne et la seconde partie externe ; et
une tête (120) positionnée au niveau de la région d'extrémité distale de la gaine, la tête définissant une ouverture (122) à proximité de la région d'extrémité distale configurée pour être couplée de manière fluidique à un tube (108) efficace pour permettre une communication fluidique entre la chambre et le tube.

2. Dispositif de collecte de fluide selon la revendication 1, dans lequel la première couche inclut un textile filé-lié présentant la première partie interne et la première partie externe et incluant du polyéthylène et du polypropylène.

3. Dispositif de collecte de fluide selon la revendication 2, dans lequel la première partie interne inclut un polypropylène non tissé et la première partie externe inclut un film microporeux de polyéthylène.

4. Dispositif de collecte de fluide selon la revendication 2, dans lequel la première partie interne inclut un matériau de polypropylène non tissé, la première partie externe inclut un matériau de polypropylène non tissé, et la première couche inclut une première partie intermédiaire incluant du polyéthylène entre la première partie interne et la première partie externe.

5. Dispositif de collecte de fluide selon la revendication 1, dans lequel la première partie interne inclut un matériau de polyester non tissé et la première partie externe inclut un matériau de polyéthylène.

6. Dispositif de collecte de fluide selon la revendication 1, dans lequel la première couche inclut un textile filé-lié, la première partie interne inclut un matériau de cellulose et la première partie externe inclut du polyéthylène.

7. Dispositif de collecte de fluide selon la revendication 1, dans lequel la première partie interne et la première partie externe incluent du polypropylène filé-lié, et la première couche inclut en outre une partie intermédiaire incluant du polypropylène soufflé par fusion entre la première partie interne et la première partie externe.

8. Dispositif de collecte de fluide selon la revendication 1, dans lequel la première partie interne inclut du polypropylène filé-lié et la première partie externe inclut du polyéthylène non tissé, la première couche incluant en outre une première partie intermédiaire incluant du polypropylène soufflé par fusion adjacente à la première partie interne,
une deuxième partie intermédiaire incluant du polypropylène filé-lié adjacente à la première partie intermédiaire, et une troisième partie intermédiaire incluant du polypropylène soufflé par fusion entre la deuxième partie intermédiaire et la première partie externe.

9. Dispositif de collecte de fluide selon la revendication 1, dans lequel la première partie interne inclut du polypropylène filé-lié et la première partie externe inclut du polyéthylène non tissé, la première couche incluant en outre une première partie intermédiaire incluant du polypropylène soufflé par fusion adjacente à la première partie interne et une deuxième partie intermédiaire incluant du polypropylène filé-lié entre la première partie intermédiaire et la première partie externe.

10. Dispositif de collecte de fluide selon l'une quelconque des revendications 1 à 9, dans lequel la première couche est configurée pour fournir une évacuation à la chambre quand un vide d'environ 0 mmHg à environ 20 mmHg est attiré sur la chambre par l'ouverture.

11. Dispositif de collecte de fluide selon l'une quelconque des revendications 1 à 10,
dans lequel la première couche inclut une couche poreuse présentant des pores d'environ 0,1 µm à environ 3 µm ; ou
dans lequel la seconde couche inclut un textile d'espacement incluant la seconde partie interne, la seconde partie externe et la partie intermédiaire reliant la seconde partie interne et la seconde partie externe ; dans lequel le textile d'espacement de la seconde couche inclut un textile d'espacement en polyester ; ou
dans lequel la seconde partie interne inclut une partie maillée interne présentant des trous d'une première taille et la seconde partie externe inclut une partie maillée externe présentant des trous d'une seconde taille plus petits que les trous de la première taille ; ou dans lequel la seconde partie interne inclut un textile de polyester
et la seconde partie externe inclut un maillage de polyester ; ou dans lequel la seconde partie interne inclut un textile de coton et la seconde partie externe inclut un maillage de polyester ; ou dans lequel le textile d'espacement présente une densité d'environ 190 gsm à environ 260 gsm.

12. Dispositif de collecte de fluide selon la revendication 1, dans lequel la seconde couche inclut un textile tricoté de polyester-élasthanne et au moins l'un parmi :
la seconde partie interne inclut le textile tricoté de polyester-élasthanne et la seconde
partie externe inclut le textile tricoté de polyester-élasthanne ; ou
la seconde partie interne inclut un textile de coton et la seconde partie externe inclut le textile tricoté de polyester-élasthanne ; ou
le textile tricoté de polyester-élasthanne présente une densité d'environ 250 gsm à environ 380 gsm.

13. Dispositif de collecte de fluide selon l'une quelconque des revendications 1 à 12, dans lequel la seconde couche présente une épaisseur d'environ 1,0 mm à environ 6,0 mm.

14. Dispositif de collecte de fluide selon l'une quelconque des revendications 1 à 13, dans lequel la seconde partie interne de la seconde couche délimite au moins partiellement la chambre.

15. Dispositif de collecte de fluide selon l'une quelconque des revendications 1 à 14, dans lequel la première partie externe de la première couche délimite au moins partiellement une surface externe de la gaine.
